Europäisches Patentamt

(19) European Patent Office

Office européen des brevets

(11) Publication number: **0 241 971**
**B1**

## (12) EUROPEAN PATENT SPECIFICATION

(45) Date of publication of the patent specification:
**22.11.90**

(21) Application number: **87200566.5**

(22) Date of filing: **25.03.87**

(51) Int. Cl.⁵: **A61F 6/08, A61K 9/02**

(54) Method for manufacturing a medicated disposable pessary and medicated disposable pessary.

(30) Priority: **11.04.86 NL 8600929**

(43) Date of publication of application:
**21.10.87 Bulletin 87/43**

(45) Publication of the grant of the patent:
**22.11.90 Bulletin 90/47**

(84) Designated Contracting States:
**AT BE CH DE ES FR GB IT LI NL SE**

(56) References cited:
**EP-A- 0 094 695**
**GB-A- 2 096 466**
**US-A- 4 093 490**
**US-A- 4 369 773**
**US-A- 4 393 871**
**US-A- 4 427 477**

(73) Proprietor: **Fundatech S.A., 1, rue Robert-de-Traz Case postale 29 Champel, CH-1211 Genève - 12(CH)**

(72) Inventor: **Spits, Marc, 125 St. Odilialaan, B-3590 Achel(BE)**

(74) Representative: **Kupecz, Arpad et al, Octrooibureau Los en Stigter B.V. Postbox 20052, NL-1000 HB Amsterdam(NL)**

ACTORUM AG

## Description

The present invention relates to a method for manufacturing a medicated disposable pessary consisting of a porous spongy synthetic material and a resilient synthetic ring, the method comprising curing the ring together with said porous spngy synthetic material followed by impregnating the obtained pessary with a solution of at least one medication.

Such a method for manufacturing a medicated disposable pessary is disclosed in EP-A 0 094 695.

Furthermore reference is made to US-A 4 427 477 for a method for manufacturing a cervical contraceptive disphragm assembly.

Finally reference is made to US patent specification 4 393 871. According to this known method, at first the porous spongy material is prepared by mixing a polyurethane prepolymer with an aqueous solution of for instance a spermicide/surfactant. After mixing, the blend is poured into a polymeric cavity mould, where it begins to foam as the hydroxyl groups from the water react with the isocyanate groups to form polyurethane. The special feature of this known method is the obtaining of a synergistic combination of a spermicide serving as surfactant and a polyurethane prepolymer. The pessary is provided with a removal means, which is secured to the polyurethane during foaming.

The known pessary thus obtained has the important disadvantage of not being anchorable in the vagina, thus being subject to displacement, which involves the risk for the woman of getting pregnant.

A further disadvantage is that the pessary produced by the prior art method is voluminous.

Finally said pessary should be wetted prior to insertion into the vagina.

The present invention is directed to a method, in which the above mentioned disadvantages are efficiently removed.

To this end the present invention provides a method for manufacturing a medicated disposable pessary consisting of a porous spongy synthetic material and a resilient synthetic ring, the method comprising curing the ring together with said porous spongy synthetic material, followed by impregnating the obtained pessary with a solution of at least one medication, characterized in that said porous spongy synthetic material with a thickness of 0,5–1,0 cm is compressed into the desired shape, wherein the thickness of the porous spongy material is reduced with 10–20% with respect to its original thickness prior to its curing.

It will be appreciated, that by this remarkable reduction of thickness of the porous spongy synthetic material an important decrease in volume of the pessary is obtained, having as favourable effect that the desired properties of foam, such as softness and the desired absorption respectively release of the medications, are retained.

The resilient synthetic ring has such a diameter as to fix the pessary in the vagina, with the pessary blocking the cervix suitably and permanently, so that the chance of spermatozoa reaching the uterus is reduced to a minimum.

The diameter of the resilient synthetic ring may vary in dependence of the size of the cervix and may amount to 5-10 cm.

Usually, the compression of the porous spongy synthetic material is carried out at a temperature of 180-220°C and at a small pressure of less than 10 kg/cm².

The curing, which secures the synthetic ring to the material compressed into shape, is conducted at 180-220°C and at a pressure of 70-115 kg/cm².

According to the invention it has been found, that the curing of the invention provides a good adherence between the resilient synthetic ring and the porous material, so that even after a prolonged stay of the pessary in the vagina a release of the ring does not occur.

According to the present invention, it is preferred to use as porous spongy synthetic material a thermically cross-linked polyester polyurethane foam having a controlled pore size. The number of pores per linear centimeter amounts to 30-50.

However, also other appropriate porous synthetic spongy materials may be used.

According to the present invention good results are obtained when the resilient synthetic ring consists of a material having a melting point lower than the porous spongy synthetic material.

A preferred pessary is obtained, according to the invention, by using as resilient synthetic ring an ethylene vinyl acetate ring and as porous spongy synthetic material thermically cross-linked polyester polyurethane foam.

Finally, the obtained pessary is impregnated with a medication, preferably spermicides and/or other substances, after which the pessary thus impregnated is packed in an air/liquid tight unitary packing, in which packing it is traded.

In addition to spermicides, such as nonoxynol-9, p-methanyl phenyl polyoxyethylene ether, polyoxyethylene oxypropylene stearate etc., other substances may be used, for instance fungicides, bactericides, virucides etc.

A specially suitable spermicide is a mixture of nonoxynol-9 and chlorhexidine gluconate.

The invention will be described in more detail by the following non-restrictive example.

### EXAMPLE

A suitable disposable pessary according to the invention is manufactured by compressing a thermically cross-linked polyester polyurethane foam with a thickness of approximately 0,75 cm into the desired shape at 200°C and at a pressure of 5 kg/cm², producing a 0,075 cm thick foam material. Then a resilient ethylene vinyl acetate ring of proper diameter, for example 6 cm, is secured to the compressed spongy synthetic material by curing at 200°C and at a pressure of 100 kg/cm².

Next the pessary thus obtained is impregnated with a solution of nonoxynol-9 and chlorhexidine gluconate as spermicide. Such a solution contains 3,3 ml of sterile water, 0,48 ml of nonoxynol-9 and 0,2 ml of 20% chlorhexidine gluconate.

Finally, the pessary thus impregnated is packed in an air/liquid tight unitary packing. This packed pessary of the present invention is ready for distribution in a pharmacy, druggist's shop, etc.

**Claims**

1. A method for manufacturing a medicated disposable pessary, consisting of a porous spongy synthetic material and a resilient synthetic ring, the method comprising curing the ring together with said porous spongy synthetic material, followed by impregnating the obtained pessary with a solution of at least one medication, characterized in that said porous spongy synthetic material with a thickness of 0,5–1,0 cm is compressed into the desired shape, wherein the thickness of the porous spongy material is reduced with 10–20% with respect to its original thickness prior to its curing.

2. The method of claim 1, characterized in that the compression of the porous spongy synthetic material is carried out at a temperature of 180-220°C and at a small pressure of less than 10 kg/cm$^2$.

3. The method of claims 1 or 2, characterized in that the curing is carried out at a temperature of 180-220°C and at a pressure of 70-115 kg/cm$^2$.

4. The method of claims 1–3, characterized in that the porous spongy synthetic material with relatively large thickness is a thermically cross-linked polyester polyurethane foam having a controlled pore size.

5. The method of claims 1–3, characterized in that the resilient synthetic ring consists of a material having a melting point lower than the porous spongy synthetic material.

6. The method of claim 5, characterized in that the resilient synthetic ring consists of ethylene vinyl acetate, with the porous spongy synthetic material being thermically cross-linked polyester polyurethane foam.

7. The method of claims 1–6, characterized in that the pessary obtained is impregnated with spermicidal and/or other substances, after which it is packed in air/liquid tight unitary packings.

**Patentansprüche**

1. Verfahren zur Herstellung eines antiseptischen Einweg-Pessars, welches aus einem porösen schwammartigen synthetischen Material und einem elastischen synthetischen Ring hergestellt wird, wobei das Verfahren folgende Schritte aufweist: Der Ring wird mit dem porösen schwammartigen synthetischen Material zusammenvulkanisiert und dann wird das Pessar mit einer Lösung von mindestens einem Antiseptikum imprägniert, dadurch gekennzeichnet, daß das poröse schwammartige synthetische Material mit einer Dicke von 0,5 bis 1,0 cm in die gewünschte Form gepreßt wird, wobei die Dicke des porösen schwammartigen Materials auf 10 bis 20% seiner vor der Vulkanisation ursprünglichen Dicke reduziert wird.

2. Verfahren nach Anspruch 1, dadurch gekennzeichnet, daß das poröse schwammartige synthetische Material bei einer Temperatur von 180 bis 220°C und bei einem geringen Druck von weniger als 10 kg/cm$^2$ gepreßt wird.

3. Verfahren nach den Ansprüchen 1 oder 2, dadurch gekennzeichnet, daß das Vulkanisieren bei einer Temperatur von 180 bis 220°C und einem Druck von 70 bis 115 kg/cm$^2$ durchgeführt wird.

4. Verfahren nach den Ansprüchen 1 bis 3, dadurch gekennzeichnet, daß als poröses schwammartiges synthetisches Material ein thermisch vernetzter Polyester-Polyurethanschaum mit vorgegebener Porengröße verwendet wird.

5. Verfahren nach den Ansprüchen 1 bis 3 dadurch gekennzeichnet, daß für den elastischen synthetischen Ring ein Material verwendet wird, dessen Schmelzpunkt niedriger als der des porösen schwammartigen synthetischen Materials liegt.

6. Verfahren nach Anspruch 5, dadurch gekennzeichnet, daß der elastische synthetische Ring aus einem Ethylen-Vinyl-Acetat hergestellt wird, wobei als poröses schwammartiges synthetisches Material ein thermisch vernetzter Polyester-Polyurethanschaum verwendet wird.

7. Verfahren nach den Ansprüchen 1 bis 6, dadurch gekennzeichnet, daß das Pessar mit Spermiziden und/oder anderen Substanzen imprägniert wird, wonach es in luft-/flüssigkeitsdichte Einheitspackungen gepackt wird.

**Revendications**

1. Procédé de fabrication d'un pessaire médicamenté à jeter après usage, constitué d'une matière synthétique spongieuse poreuse et d'un corps annulaire synthétique élastique, procédé consistant à traiter thermiquement le corps annulaire conjointement avec ladite matière synthétique spongieuse poreuse, puis à imprégner le pessaire obtenu d'une solution d'au moins une médication, caractérisé en ce que ladite matière synthétique spongieuse poreuse ayant une épaisseur de 0,5 à 1,0 cm est comprimée à la forme désirée, de manière à réduire l'épaisseur de cette matière de 10 à 20% par rapport à son épaisseur initiale avant son traitement thermique.

2. Procédé suivant la revendication 1, caractérisé en ce que la compression de la matière synthétique spongieuse poreuse est effectuée à une température de 180 à 220°C et sous une faible pression inférieure à 10 kg/cm$^2$.

3. Procédé suivant la revendication 1 ou 2, caractérisé en ce que le traitement thermique est effectué à une température de 180 à 220°C et sous une pression de 70 à 115 kg/cm$^2$.

4. Procédé suivant les revendications 1 à 3, caractérisé en ce que la matière synthétique spongieuse poreuse est une mousse de polyester-polyuréthanne réticulée thermiquement dont les pores ont des diamètres réglés.

5. Procédé suivant les revendications 1 à 3, caractérisé en ce que le corps annulaire synthétique élastique est constitué d'une matière ayant un point de fusion inférieur à celui de la matière synthétique spongieuse poreuse.

6. Procédé suivant la revendication 5, caractérisé en ce que le corps annulaire synthétique élastique est en éthylène-acétate de vinyle, la matière synthétique spongieuse poreuse étant une mousse de polyester-polyuréthanne réticulée thermiquement.

7. Procédé suivant les revendications 1 à 6, caractérisé en ce que le pessaire obtenu est imprégné d'une substance spermicide et/ou d'autres substances, après quoi il est conditionné sous emballages unitaires étanches à l'air et aux liquides.